Europäisches Patentamt

European Patent Office

Office européen des brevets

(19)

(11) Veröffentlichungsnummer: **0 479 180 A2**

(12) **EUROPÄISCHE PATENTANMELDUNG**

(21) Anmeldenummer: **91116635.3**

(22) Anmeldetag: **30.09.91**

(51) Int. Cl.[5]: **C12N 15/11**, C12N 5/10,
A01H 5/00, A61K 31/70,
//C12N15/55,C12N9/22

(30) Priorität: **05.10.90 DE 4031543**

(43) Veröffentlichungstag der Anmeldung:
**08.04.92 Patentblatt 92/15**

(84) Benannte Vertragsstaaten:
**BE CH DE ES FR GB GR IT LI NL**

(71) Anmelder: **HOECHST AKTIENGESELLSCHAFT
Postfach 80 03 20
W-6230 Frankfurt am Main 80(DE)**

(72) Erfinder: **Eckes, Peter, Dr.
Am Flachsland 18
W-6233 Kelkheim (Taunus)(DE)**
Erfinder: **Schneider, Rudolf, Dr.
Feldbergstrasse 98
W-6233 Kelkheim (Taunus)(DE)**

(54) **Virusresistente Pflanzen, Verfahren zu ihrer Herstellung.**

(57)

Die Erfindung betrifft Gene zur Herstellung virusresistenter Pflanzen

Das Gen kodiert für eine Sequenz, die als Initiationssequenz zur Synthese einer komplementären RNA dienen kann und die in Folge verknüpft ist mit einer antisense-Sequenz, die in sense Orientierung eine cap unabhängige Translation ermöglicht sowie mit einer antisense Sequenz, die in sense Orientierung für ein für den Stoffwechsel einer Pflanze destruktives Protein kodiert.
Nachdem ein Virus die Zelle befallen hat, wird die RNA mittels der viralen Replikase oder durch eine Replikasefunktion, die über einen anderen Mechanismus in der Zelle aktiviert wurde, in eine komplementäre RNA umgeschrieben und die nun in sense Orientierung vorliegende Information für ein zelldestruktives Enzym wird abgelesen.

EP 0 479 180 A2

Rank Xerox (UK) Business Services

Phytopathogene Viren parasitieren vom Stoffwechsel ihrer Wirtspflanzen und können je nach befallener Spezies und Sorte erhebliche Schäden verursachen. Die Viren gelangen in den allermeisten Fällen über Wunden oder mittels Überträgern in das Pflanzengewebe. Tierische Überträger, die mit Viren infiziert sind, geben beim Einstich ins Pflanzengewebe oder bei der Fraßtätigkeit Viren ab. Die Übertragung eines bestimmten Virustyps durch ein Insekt ist vermutlich hauptsächlich durch das "coat"-Protein des Virus beeinflußt (Chen, B. et al., Journal of General Virology 71, 939-944, 1990). Auf welche Weise auch immer Viren in pflanzliches Gewebe gelangen, sie müssen, um sich vermehren zu können, letzlich durch intakte Zellmembranen in die Wirtszellen eindringen. Zellen mit beschädigtem Membransystem sterben in der Regel sehr schnell ab. Wie die Viren die Zellmembranen passieren, ist noch nicht endgültig geklärt. So ist denkbar, daß die Pflanzenzellen selbst in einem Pinozytose ähnlichen Vorgang das Virus aktiv aufnehmen. Andere Überlegungen postulieren die Beteiligung von Rezeptoren auf der Pflanzenoberfläche, die mit Virusproteinen in Wechselwirkung treten. Für diese Auffassung gibt es einige Hinweise, aber noch keine endgültige Lösung. So konnte in einem Versuch gezeigt werden, daß eine Mischung von Tabak Mosaik Virus (TMV) und coat protein desselben Virus auf Nicotiana tabacum var. Xanthi (Holoubek, V. Nature 203, 499-501, 1964) oder Chenopodium amaranticolor (Novikov, V.K. et al., Virology 41, 101-107, 1970) weniger Läsionen verursachte als ein Virus alleine. Heterologes Protein von BSMV (Barley Stunt Mosaic Virus) dagegen zeigte keinen Effekt (Novikov, V.K. et al., Virology 41, 101-107, 1970).

Die Nukleinsäure besteht bei phytopathogenen Viren meistens aus RNA und nur selten aus DNA. Die Viruspartikel, die im wesentlichen aus Nukleinsäure, umgeben von einer Proteinhülle (Capsid) aus Untereinheiten bestehen, haben verschiedenartige Formen. Am häufigsten findet man stäbchenförmige (elongiert) mit helikaler Symmetrie wie z.B. TMV und isometrische (sphärisch) mit ikosaedrischer Symmetrie z.B. Cucumber Mosaic Virus (CMV) und Geminiviren. Die Viren unterscheiden sich hinsichtlich Form, Nukleinsäuregehalt, Nukleinsäureform (ein- oder zweisträngig), Molekulargewicht der Nukleinsäure sowie hinsichtlich der Wirtspflanzen und der Überträger, die sie auf Wirtspflanzen übertragen.

Ein relativ gut charakterisiertes und in Gemüsepflanzen ökonomisch bedeutendes Virussystem ist das Gurkenmosaikvirus (CMV; Cucumber Mosaic Virus). Das Virus besteht aus 3 funktionellen Stücken einzelsträngiger RNA, die in 3 verschiedene icosaedrische Partikel mit 28 nm Durchmesser verpackt werden, und zu gleichen Raten sedimentieren (tripartite Virus) (CMI/AAB Decriptions of Plant Viruses, No. 213 (No. 1 revised), July 1979). Das Virus verursacht neben anderen Symptomen Mosaikbildung, z.B. auf Gurken und anderen Cucurbitaceen, sowie Nekrosen und Fäulnisbildung, z.B. auf Spinat und Tomaten. Der Wirtsbereich ist ungewöhnlich groß. So werden Nutz- und Zierpflanzen, auch Bäume und Büsche aus über 40 Familien befallen (CMI/AAB Decriptions of Plant Viruses, No. 213 (No. 1 revised), July 1979).

Es gibt verschiedene Ansätze, virusresistente Pflanzen zu erhalten. Durch Expression von coat protein verschiedener Viren gelang es, Symptome des jeweiligen Virus auf der Pflanze abzuschwächen und die Virusvermehrung zu behindern. Ausgegangen war dieser Ansatz von der Beobachtung, daß eine Virusinfektion sehr viel schwächer ist, wenn die Pflanze mit einem verwandten Virus vorher infiziert wurde (Hamilton, R.I. in Plant Disease V (Horsfall, J.G. and Cowling, F.B., eds), pp. 279-303, Academic Press (1980). Mit TMV gelang es, in einem in vitro System zu zeigen, daß die Freisetzung von RNA aus TMV-Teilchen und die anschließende Translation behindert wird, wenn einem Extrakt coat protein zugesetzt wird (Wilson, T.M.A. et al., Virology 145, 346-349, 1985). Die Überexpression von TMV coat protein in transgenem Tabak führte gleichfalls zu einer starken Abschwächung der TMV Infektion (Powell-Abel, P. et al., Science 232, 738-743, 1986). Ähnliche Ergebnisse ergaben sich bei einer Überexpression der coat protein Gene von Alfalfa Mosaic Virus (Tumer, N.E. et al., EMBO J. 6, 1181-1188, 1987; Loesch-Fries, L.S. et al., EMBO J. 6, 1845-1851, 1987), Kartoffel Virus X (Hemenway, C. et al., EMBO J. 7, 1273-1280, 1988).

Eine weitere Möglichkeit resistente Pflanzen zu erhalten, beinhaltet die Transkription von antisense RNA gegen das Pflanzenvirusgenom. Zugrundegelegt wird dabei die Annahme, daß die exprimierte antisense RNA mit der viralen RNA ein "Annealing" vollzieht und dadurch die Expression der Virus-RNA behindert. Es konnte für Kartoffel Virus X und Cucumber Mosaik Virus gezeigt werden, daß ein Schutz der Pflanzen gegen Virusinfektion besteht, wenn antisense RNA exprimiert wird, allerdings mit der Einschränkung, daß die verwendeten Viruskonzentration beim Testen sehr niedrig sein müssen (Hemenway, C. et al., EMBO J. 7, 1273-1280, 1988; Cuozzo, M. et al., Bio/Technology 6, 549-558, 1988). Für Tobacco Rattle Virus gelang es nicht, durch antisense Expression eine Virusresistenz in transgenen Pflanzen zu erzeugen (Baulcombe et al. in Plant Resistance to Viruses (CIBA Foundation Symposium 133) (Evered, D. and Harnett, S., eds), pp. 170-184, John Wiley (1987).

Für einige Virussysteme sind Satelliten-RNA Moleküle beschrieben, die eine Abschwächung der Virussymptome bewirken. Satelliten RNA Moleküle sind in der Regel klein (300-500 Basen) und hängen bei der Vermehrung vollständig von einem Helfer Virus ab (Francki, R.I.B., Ann u. Rev. Microbiol. 39, 151-174, 1985). Die Satelliten RNAs sind nicht immer in einer viralen Kultur anwesend. Mit der DNA einer CMV Satelliten RNA in Agrobakterien gelang es transgene Pflanzen zu konstruieren, die Satelliten RNA exprimierten (Baulcombe, D.C. et al., Nature 321, 446-449, 1986; Harrison, B.D. et al., Nature 328, 799-802, 1987). Die transgenen Pflanzen zeigten eine verminderte Virusproduktion und Abschwächung der Symptome im Test mit CMV-Partikeln (Harrison, B.D. et al., Nature 328, 799-802, 1987).

Eine weitere Alternative zur Erzeugung von Virusresistenz in Pflanzen, die von der Züchtung schon immer praktiziert wurde, ist die Auswahl einer pflanzencodierten Virusresistenz und Übertragung auf interessante Sorten. Die Molekularbiologie eröffnet hierbei die Möglichkeit, Gene von Spezies zu Spezies zu transferieren und Bereiche zu erschließen, die vorher nicht zugänglich waren.

Es wurde nun eine neuer Ansatz zur Erzeugung virusresistenter Pflanzen gefunden. Ausgegangen wird dabei von einer RNA-Sequenz, die als Initiationssequenz zur Synthese einer komplementären RNA dienen kann. In Frage kommen dafür alle Sequenzen, die es einer Replikase erlauben eine komplementäre RNA-Synthese zu initiieren. Die ursprüngliche Virussequenz sollte so modifiziert werden, daß in der umgeschriebenen, komplementären RNA kein 5'-AUG-3' auftritt. Es wird so sichergestellt, daß das AUG der im umgeschriebenen Strang in sense vorliegenden Funktion als erstes Startcodon erkannt wird. Sollte keine entsprechende Basensequenz in der ursprünglichen RNA vorhanden sein, dann kann diese auch unverändert verwendet werden. Das gleiche gilt für den Fall, daß sich ein vorhandenes AUG im umgeschriebenen Strang als nicht störend für die Translation der gewünschten Funktion erweist. Bevorzugt wird aber die modifizierte Virussequenz verwendet.

An das 5'-Ende dieser Sequenz ist eine antisense-Sequenz gekoppelt, die in sense Orientierung eine cap unabhängige Translation ermöglicht. Anschließend folgt die antisense Sequenz eines für den Stoffwechsel der Pflanze destruktiven Gens. Das konstruierte Gen kann mit der Sequenz für das destruktive Gen enden, oder das 5'-Ende besteht aus dem nicht kodierenden 5'-Ende eines RNA Virus, es kann aber auch andere Sequenzen mit Funktionalität enthalten.

Das obengenannte 3'-Ende einer RNA zur Initiation der komplementären RNA kann auch mit einem beliebigen Gen in sense Orientierung verbunden sein. Wenn ein Virus RNA 5'-Ende oder eine interne Virus-RNA-Sequenz mit derselben Funktion, nämlich Initiationsstart zum Umschreiben in komplementäre RNA, bekannt wäre, könnte dieses mit den entsprechenden Modifizierungen in den einzufügenden Gensequenzen, dieselbe Rolle wie das beschriebene 3'-Ende spielen.

Eine so beschriebene RNA wird durch ein passendes DNA Stück im Pflanzengenom von der Pflanze konstitutiv oder auch induziert exprimiert und hat in der nicht infizierten Pflanze keine Funktion. Wenn ein Viruspartikel die Zelle befällt, wird durch das miteingebrachte Replikase-Enzym die RNA in komplementäre RNA umgeschrieben, die nun wiederum die Translation eines zellzerstörenden Enzyms ermöglicht. Bei geeigneter Wahl der Funktion wird die Zelle zerstört und das Virus an der weiteren Ausbreitung gehindert. Nicht infizierte Pflanzenzellen zeigen diese Reaktion nicht.

Die Erfindung betrifft somit

1. Ein Gen kodierend auf RNA-Ebene für eine Sequenz, die als Initiationssequenz zur Synthese einer komplementären RNA dienen kann und die in Folge gekoppelt ist an eine antisense-Sequenz, die in sense Orientierung eine cap unabhängige Translation ermöglicht, sowie mit einer antisense RNA Sequenz, die in sense Orientierung für ein für den Stoffwechsel einer Pflanze destruktives Protein kodiert.

Dieses Gen muß eine RNA exprimieren, die durch eine Replikasefunktion aus einem Viruspartikel freigesetzt, durch ein Virus erzeugt oder über einen anderen Mechanismus in der Zelle aktiviert, in die komplementäre Sequenz umgeschrieben werden kann.

Gleichzeitig muß aus der umgeschriebenen, komplementären Form nach der Translation die beabsichtigte Funktion erzeugt werden können.

2. Wirtszellen, die das unter 1. charakterisierte Gen enthalten.

3. Die Verwendung der durch das unter 1. charakterisierte Gen kodierten RNA als Vorstufe zu einem antiviralen Agens.

Im folgenden wird die Erfindung detailliert beschrieben, insbesondere in ihren bevorzugten Ausführungsformen. Ferner wird die Erfindung durch den Inhalt der Ansprüche bestimmt.

Zur Synthese des erfindungsgemäßen Gens werden zuerst die genannten Teilstücke bereitgestellt, die dann über Linker miteinander verknüpft werden.

Die Synthese des ersten DNA-Teilstücks erfolgt vorteilhaft auf Basis der 3'-Sequenz eines RNA-Virus. Bevorzugt wird RNA aus pflanzenpathogenen Viren verwendet, insbesondere aus Cucumber-Mosaic-Virus, Brom-Mosaic-Virus, Tabak-Mosaik-Virus, Alfalfa Mosaic Virus sowie aus Cowpea Mosaic Virus.

Für die verwendete 3'-Sequenz ist wesentliche Voraussetzung, daß sie als Initiationssequenz zur Synthese einer (-)RNA dienen kann oder im Falle von (-)RNA Viren zur Synthese des (+)RNA Stranges, also jeweils für die komplementäre RNA-Sequenz.

Die ursprüngliche Virussequenz wird an allen 5'-CAU-3'-Stellen der zu exprimierenden RNA so verändert, daß in der umgeschriebenen Form des Moleküls keine 5'-AUG-3'-Sequenzen auftreten. Die Sequenz muß so geändert werden, daß die Funktion des 3'-Endes als Replikase Promotor erhalten bleibt. Die Änderung der Sequenz wird vorgenommen durch Einführung einer Punktmutation an einer oder mehreren Stellen.

Wenn sichergestellt ist, daß das 3'-Ende der (-)RNA eines (+)RNA Virus oder entsprechend das 3'-Ende der (+)RNA eines (-)RNA Virus als Initiationsort der Replikase erkannt wird und zum Umschreiben des RNA-Moleküls führt, kann auch mit der 3'-Sequenz der (-)RNA des (+)RNA Virus oder der 3'-Sequenz der (+)RNA des (-)Virus gearbeitet werden, wobei auch hier am vorteilhaftesten die Sequenz so modifiziert werden muß, daß in der umgeschriebenen RNA vor dem Startcodon der zu exprimierenden Funktion kein AUG mehr auftritt.

Vorteilhaft wird das DNA-Teilstück mit Hilfe der RNA des Cucumber- bzw. Brom-Mosaic-Virus synthetisiert, insbesondere ausgehend von den letzten 200 bis 400 Nukleotiden, bevorzugt den letzten 280-320 Nukleotiden des 3'-Endes.

Die von Gould, A.R. in Eur. J. Biochem. 126, 217-226 (1982) publizierte RNA Sequenz des Cucumber Mosaic Virus wird mit gutem Erfolg als Vorlage zur DNA Synthese verwendet.

Das zweite DNA-Teilstück wird vorteilhaft ausgehend von der cap independent translation sequence RNA des Tomato Etch Virus synthetisiert (Carrington, J.C. et al., J. Vir. 64 1590-1597, 1990). Die ersten 150-200 Nukleotide des 5'-Endes werden bevorzugt hierfür eingesetzt.

Es können auch alle anderen RNA-Sequenzen aus anderen Viren verwendet werden, sofern sie in der sense Orientierung eine cap unabhängige Translation ermöglichen. Insbesondere bevorzugt wird die von Allison, R. Virology 154, 9-20, (1986) veröffentlichte RNA Sequenz als Basis zur DNA Synthese eingesetzt. Die DNA wird so synthetisiert, daß sie auf RNA-Ebene für die antisense RNA der "cap independent translation sequence" kodiert.

Das dritte Teilstück besteht aus dem Gen, das für ein zelldestruktives Enzym kodiert, bevorzugt für Proteasen, DNasen, RNasen, Replikasen, Lipasen sowie Enzymen, die die Membrankonformation störend beeinflussen. Bevorzugt werden Gene verwendet, die für RNAsen kodieren. Vorteilhaft wird die von Ikehara, M. in Proc. Natl. Acad. Sci. 83, 4695 (1986) publizierte DNA-Sequenz eingesetzt. Auch hier erfolgt die Synthese derart, daß die DNA auf RNA-Ebene für die antisense Sequenz kodiert.

Nach Ligation der Teilstücke wird das Gen transkribiert, ist aber nicht funktionell. Das Gen wird erst durch eine hinzukommende Replikase (des Virus) durch Umschreiben in die komplementäre RNA aktiviert, wobei die antisense in sense RNA umgeformt wird. Die Replikase kann durch eine analoge Funktion ersetzt werden.

Ferner kann RNA kodierend für das 5'-Ende einer Virus RNA angehängt werden, das in seiner (-) Form die Funktionalität des oben beschriebenen 3'-Endes besitzt. Diese 5'RNA Sequenz kann aus den obengenannten Quellen isoliert werden.

Die gegebene Information für das 3'-RNA-Ende gilt also entsprechend für das 5'-RNA-Ende.

Die zusammengesetzten Oligonukleotide werden mit Hilfe der Vektoren pUC19, pUC18 oder pBluescript (Stratagene, Heidelberg, Product Information) kloniert und sequenziert.

Das bestätigte Oligonukleotid wird in einen intermediären Plasmidvektor zwischen einen in Pflanzen wirksamen Promotor und Terminator kloniert. Bevorzugt wird der Vektor pDH51 (Pietrzak, M. et al., NAR 14, 5857, (1986)) mit einem 35S-Promotor und Terminator verwendet.

Als Terminator kann ebenfalls jede Sequenz gewählt werden, die ein 3'-Ende erzeugt, das als Substrat für die Replikase eingesetzt werden kann und zu einer funktionellen, komplementären RNA führt. Möglich wäre es z.B. den Terminator eines Polymerase I- oder Polymerase III-Gens zu verwenden. Ein anderer denkbarer Ansatz ist die Verwendung einer Ribozymsubstratstelle als unmittelbares Ende der Virussequenz, auf die eine Ribozymfunktion folgen müßte, mit der vorangehenden Sequenz als Substrat.

Nach anschließender Transformation von E. coli, wie z.B. E. coli MG 1061, DH1, DK1 oder XL-1, werden positive Klone nach an sich bekannten Methoden (Maniatis et al., Molecular Cloning: A Laboratory Manual, Cold Spring Harbor Laboratory, Cold Spring Harbor, New York, 1982), wie Plasmidminipräparation und Spaltung mit einem entsprechenden Restriktionsenzym, identifiziert.

Die so erhaltenen Vektoren werden verwendet, um Pflanzen zu transformieren. Dies kann durch Techniken wie Elektroporation oder Mikroinjektion erfolgen.

Das DNA-Fragment bestehend aus Promotor, Oligonucletid und Terminator wird ebenfalls in Pflanzenvektoren subkloniert.

Als Pflanzenvektoren können z.B. pGV3850 (Zambryski, P. et al, EMBO J. 2, 2143-2150 (1983)) oder pOCA18 (Olszewski, N., NAR 16, 10765-10782, (1988)) eingesetzt werden. Vorteilhaft wird mit pOCA18 gearbeitet.

Bevorzugt wird die Kokultivierung von Protoplasten oder die Transformation von Blattstückchen mit Agrobakterien angewandt. Dazu wird das Pflanzenvektorkonstrukt durch Transformation mit gereinigter DNA oder, vermittelt über einen Helferstamm wie E. coli SM10 (Simon R. et al., Biotechnology 1, 784-791 (1983)) in Agrobakterium tumefaciens wie A281 oder C58 mit einem Ti Plasmid über ein "triparental mating" transferiert. Direkte Transformation und "triparental mating" wurden, wie in "Plant Molecular Biology Manual" (Kluwer Academic Pubishers, Dardrecht (1988)) beschrieben, durchgeführt.

Es können grundsätzlich alle Pflanzen mit den die erfindungsgemäß konstruierte DNA tragenden binären Pflanzenvektoren transformiert werden. Bevorzugt sind dikotyledone Pflanzen, insbesondere Nutzpflanzen, die Stärke, andere Kohlenhydrate, Eiweiße oder Fette in nutzbaren Mengen in ihren Organen produzieren oder speichern oder die Obst und Gemüse produzieren oder die Gewürze, Fasern und technisch verwertbare Produkte oder Arzneimittel, Farbstoffe oder Wachse liefern und ebenfalls Futterpflanzen. Als Beispiel sollen Tomate, Erdbeere, Avocado, sowie Pflanzen, die tropische Früchte tragen, z.B. Papaya, Mango, aber auch Birne, Apfel, Nektarine, Aprikose oder Pfirsich genannt werden. Ferner sollen als zu transformierende Pflanzen beispielhaft alle Getreidearten, Raps, Rübsen, Kartoffeln, Sojabohne, Baumwolle, Mais, Zuckerrübe oder Sonnenblume angeführt werden. Die transformierten Zellen werden mit Hilfe eines Selektionsmediums selektiert, zu einem Kallus herangezüchtet und auf einem entsprechenden Medium zur Pflanze regeneriert (Shain et al., Theor. appl. Genet. 72, 770-770 (1986); Masson, J. et al., Plant Science 53, 167-176 (1987), Zhan et al., Plant Mol. Biol. 11, 551-559 (1988); McGranaham et al., Bio/Technology 6, 800-804 (1988) Novrate et al., Bio/Technology 7, 154-159 (1989).

Das beschriebene Wirkprinzip kann prinzipiell auch gegen andere pathogene Organismen eingesetzt werden, wenn dafür gesorgt wird, daß die virale Replikase von einem geeigneten Promotor im Pflanzengenom induziert werden kann.

Die folgenden Beispiele dienen der weiteren Erläuterung der Erfindung.

Beispiele

1. DNA-Sequenzen zur Expression einer "Suizid"-RNA

A) 3-Ende CMV nach Eur. J. Biochem. 126, 217-226, 1982 mit PstI und SphI Enden

```
                10          20          30          40
     5' CTGCAGTCCA  TCCAGCTTAC  GGCTAAAATG  GTCAGTCGTG
     3' GACGTCAGGT  AGGTCGAATG  CCGATTTTAC  CAGTCAGCAC
                50          60          70          80
        TCTTTCACAC  GCCGATGTCT  TACAAGATGT  CGAGGTACCC
        AGAAAGTGTG  CGGCTACAGA  ATGTTCTACA  GCTCCATGGG
                90         100         110         120
        TTGAAATCAC  CTCCTAGATT  TCTTCGGAAG  GGCTTCGTGA
        AACTTTAGTG  GAGGATCTAA  AGAAGCCTTC  CCGAAGCACT
               130         140         150         160
        GAAGCTCGTG  CACGGTAATA  CACTGATATT  ACCAAGAGTG
        CTTCGAGCAC  GTGCCATTAT  GTGACTATAA  TGGTTCTCAC
               170         180         190         200
        CGGGTATCGC  CTGTGGTTTT  CCACAGGTTC  TCCATAAGGA
        GCCCATAGCG  GACACCAAAA  GGTGTCCAAG  AGGTATTCCT
               210
        GACCAGCATG  C 3'
        CTGGTCGTAC  G 5'
```

B) 3'-Ende CMV nach Eur. J. Biochem. 126, 217-226, 1982 mit PstI und SphI Enden und Punktmutationen der 5'-CAT-3' Tripletts wie beschrieben 3'-GTA-5'

```
          10         20         30         40
5' CTGCAGTCCA ACCAGCTTAC GGCTAAAATG GTCAGTCGTG
3' GACGTCAGGT TGGTCGAATG CCGATTTTAC CAGTCAGCAC
          50         60         70         80
   TCTTTCACAC GCCGATGTCT TACAAGATGT CGAGGTACCC
   AGAAAGTGTG CGGCTACAGA ATGTTCTACA GCTCCATGGG
          90         100        110        120
   TTGAAATCAC CTCCTAGATT TCTTCGGAAG GGCTTCGTGA
   AACTTTAGTG GAGGATCTAA AGAAGCCTTC CCGAAGCACT
          130        140        150        160
   GAAGCTCGTG CACGGTAATA CACTGATATT ACCAAGAGTG
   CTTCGAGCAC GTGCCATTAT GTGACTATAA TGGTTCTCAC
          170        180        190        200
   CGGGTATCGC CTGTGGTTTT CCACAGGTTC TCCAAAAGGA
   GCCCATAGCG GACACCAAAA GGTGTCCAAG AGGTTTTCCT
          210
   GACCAGCATG C 3'
   CTGGTCGTAC G 5'
```

C) 3'-Ende CMV Y mit PstI und SphI Enden nach Hayakama T. et al. Gene 71, 107-114, 1988

```
          10         20         30         40
5' CTGCAGCGGG TTGTCCATCC AGCTTACGGC TAAAATGGTC
3' GACGTCGCCC AACAGGTAGG TCGAATGCCG ATTTTACCAG
          50         60         70         80
   AGTCGTGGAG AAATCTACGC CAGCAGATTT ACAAATCTCT
   TCAGCACCTC TTTAGATGCG GTCGTCTAAA TGTTTAGAGA
          90         100        110        120
   GAGGCGCCTT TGAAACCATC TCCTAGGTTT CTTCGGAAGG
   CTCCGCGGAA ACTTTGGTAG AGGATCCAAA GAAGCCTTCC
          130        140        150        160
   ACTTCGGTCC GTGTACCTCT AGCACAACGT GCTAGTTTCA
   TGAAGCCAGG CACATGGAGA TCGTGTTGCA CGATCAAAGT
          170        180        190        200
   GGGTACGGGT GCCCCCCCAC CTTCGTGGGG CCTCCAAAAG
   CCCATGCCCA CGGGGGGGTG GAAGCACCCC GGAGGTTTTC
          210
   GAGACCAGCA TGC 3'
   CTCTGGTCGT ACG 5'
```

D) 3'-Ende CMY mit PstI und SphI Enden und Punktmutation der 5'-CAT-3' Tripletts wie beschrieben 3'-GTA-5'

6

```
          10         20         30         40
5' TGCAGCGGGT TGTCCAACCA GCTTACGGCT AAAATGGTCA
3' ACGTCGCCCA ACAGGTTGGT CGAATGCCGA TTTTACCAGT
          50         60         70         80
   GTCGTGGAGA AATCTACGCC AGCAGATTTA CAAATCTCTG
   CAGCACCTCT TTAGATGCGG TCGTCTAAAT GTTTAGAGAC
          90        100        110        120
   AGGCGCCTTT GAAACCAACT CCTAGGTTTC TTCGGAAGGA
   TCCGCGGAAA CTTTGGTTGA GGATCCAAAG AAGCCTTCCT
         130        140        150        160
   CTTCGGTCCG TGTACCTCTA GCACAACGTG CTAGTTTCAG
   GAAGCCAGGC ACATGGAGAT CGTGTTGCAC GATCAAAGTC
         170        180        190        200
   GGTACGGGTG CCCCCCCACC TTCGTGGGGC CTCCAAAAGG
   CCATGCCCAC GGGGGGGTGG AAGCACCCCG GAGGTTTTCC
         210
   AGACCAGCAT GC 3'
   TCTGGTCGTA CG 5'
```

E) Nukleotidsequenz für ein RNase T1-Gen aus Proc. Natl. Acad. Sci 83, 4695-4699, 1986 mit Nco I und KpnI-Übergängen der Enden

```
          10         20         30         40
5' CCATGGATCT TCATGGCTTG CGACTACACC TGCGGCAGCA
3' GGTACCTAGA AGTACCGAAC GCTGATGTGG ACGCCGTCGT
          50         60         70         80
   ACTGCTACTC TAGCTCTGAC GTTTCTACCG CTCAGGCTGC
   TGACGATGAG ATCGAGACTG CAAAGATGGC GAGTCCGACG
          90        100        110        120
   TGGCTACCAG CTGCACGAGG ACGGCGAAAC CGTTGGCTCT
   ACCGATGGTC GACGTGCTCC TGCCGCTTTG GCAACCGAGA
         130        140        150        160
   AACTCTTACC CGCACAAATA CAACAACTAT GAGGGCTTCG
   TTGAGAATGG GCGTGTTTAT GTTGTTGATA CTCCCGAAGC
         170        180        190        200
   ACTTTAGCGT TTCTTCTCCG TACTACGAAT GGCCGATCCT
   TGAAATCGCA AAGAAGAGGC ATGATGCTTA CCGGCTAGGA
         210        220        230        240
   GTCTAGCGGC GACGTTTACT CCGGTGGTAG CCCAGGTGCT
   CAGATCGCCG CTGCAAATGA GGCCACCATC GGGTCCACGA
         250        260        270        280
   GACCGTGTAG TATTCAACGA AAACAACCAG CTCGCTGGCG
   CTGGCACATC ATAAGTTGCT TTTGTTGGTC GAGCGACCGC
         290        300        310        320
   TTATCACCCA CACCGGCGCT TCTGGCAACA ACTTTGTAGA
   AATAGTGGGT GTGGCCGCGA AGACCGTTGT TGAAACATCT
         330
   ATGCACCTAA TAGGGTACC 3'
   TACGTGGATT ATCCCATGG 5'
```

F) 5'-Ende CMV RNA3 nach Eur. J. Biochem. 126, 217-226, 1982 mit Eco RI/Bam HI und KpnI-Übergängen

```
              10          20          30          40
      5' GAATTCGGAT CCGTAATCTT ACCACTTTCT TTCACGTCGT
      3' CTTAAGCCTA GGCATTAGAA TGGTGAAAGA AAGTGCAGCA
              50          60          70          80
         GTCGCGTCAG TGACGCTGTG TGTGTGTGTG TGTTAGTTAG
         CAGCGCAGTC ACTGCGACAC ACACACACAC ACAATCAATC
              90         100         110
         TGTGTCGTGT TTAGATTACG AAGGTTGGTA CC 3'
         ACACAGCACA AATCTAATGC TTCCAACCAT GG 5'
```

G) Cap independent translation Sequenz nach Virology 154, 9-20, 1986 mit NcoI und PstI-Übergängen

```
              10          20          30          40
      5' CTGCAGTAAA TAACAAATCT CAACACAACA TATACAAAAC
      3' GACGTCATTT ATTGTTTAGA GTTGTGTTGT ATATGTTTTG
              50          60          70          80
         AAACGAATCT CAAGCAATCA AGCATTCTAC TTCTATTGCA
         TTTGCTTAGA GTTCGTTAGT TCGTAAGATG AAGATAACGT
              90         100         110         120
         GCAATTTAAA TCATTTCTTT TAAAGCAAAA GCAATTTTCT
         CGTTAAATTT AGTAAAGAAA ATTTCGTTTT CGTTAAAAGA
              130         140         150
         GAAAATTTTC ACCATTTACG AACGATAGCC ATGG 3'
         CTTTTAAAAG TGGTAAATGC TTGCTATCGG TACC 5'
```

2. Synthese der DNA zur Expression von RNA

Die Sequenzen für das beschriebene 3'-Ende des CMV-Virus für die "cap independent translation"-Struktur des TEV und das 5'-Ende werden mit einem DNA-Synthesizer (PCR-Mate 391, Fa. Applied Biosystems) hergestellt. Auch die verwendete RNAse wird synthetisch hergestellt.

3. Klonierung und Sequenzierung

3a. Klonierung der CMV 3'- und 5'-Sequenzen in pUC 19

Die CMV Sequenzen wurden wie beschrieben mit einem PstI bzw. SphI-Linker versehen. Vorzugsweise arbeitete man mit der mutierten CMV Y Sequenz, in der die potentiellen AUG-Tripletts im (-)Strang durch Punktmutationen in andere Sequenzen umgewandelt wurden. Die am Synthesizer fertiggestellten Fragmente wurden ligiert und in die PstI/SphI-Stellen von pUC 19 eingebaut.

Die Sequenzierung ergab in jedem Fall die gewünschte Sequenz. Den Polylinker aus diesem Vektor ersetzte man anschließend von PstI bis Eco RI durch einen synthetischen Polylinker mit folgender Sequenz.

```
5'-GAATTC GGTACC GAGCTC CCATGG TCTAGA CCCGGG CTGCAG-3'

3'-CTTAAG CCATGG CTCGAG GGTACC AGATCT GGGCCC GACGTC-5

    EcoRI   KpnI   SacI   NcoI   XbaI   SmaI   PstI
```

In den so umgebauten Vektor fügte man das synthetisierte Virus-5'-Ende mit einem anhängenden EcoRI/BamHI und Kpn-Linker ein. Die Sequenzierung ergab keine Fehler.

3b. Einbau des RNase-Gens und der "cap independent Translation" Sequenz

Das RNase-Gen wurde so synthetisiert, daß am 5'-Ende des ORF eine NcoI-Stelle und am 3'-Ende eine Kpn-Stelle gelegen war. Nach Einbau des RNase-Gens in die NcoI/KpnI-Stellen im Polylinker des Vektors mit den 3'- und 5'-Virussequenzen wurden positive Klone durch DNA-Minipräparationen und Restriktionsverdaus identifiziert. Die Insertion der "cap independent translation" Sequenz über die PstI und NcoI-Stellen ergänzte diesen Vektor.

4. Klonierung in pDH51

Das fertig zusammengebaute Fragment in pUC19 hatte eine Länge von etwa 1 kb. Es wurde nach Verdau der gereinigten DNA mit Bam HI und SphI aus einem Agarosegel isoliert und in einem Bam HI und SphI geschnitten pDH51 Vektor eingebaut. Der Terminatoranteil von pDH51 kann nach SphI und SacI Verdau als 0,2 kb Fragment vom Restvektor abgetrennt werden und mit Hilfe von SphI und SacI-Linkern durch einen anderen Terminator ersetzt werden. In Frage kommt dafür z.B. die im EMBO J. 6, 35-41, 1987 beschriebene Sequenz:

$$5'-TTTTGTTTTT-3'$$
$$3'-AAAACAAAAA-5'$$

aus Tabak

5. Klonierung von poCA18

Zur Expression in Pflanzen wird das zusammengebaute Gen mit dem 35 S Promotor aus pDH51 und dem angehängten Terminator in das Pflanzengenom integriert. Dazu ist eine Zwischenklonierung in den binären Pflanzenvektor pOCA 18 nötig. Das Plasmid poCA18 ist in der Veröffentlichung Olszewski, N. et al. NAR 16, 10765-10782, 1988 nacharbeitbar beschrieben. Promotor, Gen und Terminator können aus dem pDH51 Vektor eindeutig mit Hilfe von Eco RI als 1,6 kb Fragment isoliert werden. Dieses Eco RI Fragment (neben dem Vektoranteil von 2,7 kb das einzige Eco RI Fragment) wird in die singuläre Eco RI Schnittstelle von pOCA 18 eingebaut.

6. Transformation von Agrobakterien.

Der pOCA18 Vektor mit dem gewünschten Insert wurde in den Agrobakterienstamm A28 (ATCC37349, USA) überführt. Dies geschieht durch ein "Triparental mating" mit Hilfe des E. coli Stammes SM10 (Simon, R. et al., Bio/Technology 1, 784-791, 1983). Gleiche Mengen der Bakterien werden dazu über Nacht auf einen Filter gegeben, mit $\overline{2}$ ml 10 mM MgSO$_4$ abgespült und in 100 $\mu$l Mengen auf YEB-Medium ($\triangleq$ 1 % Yeast Extract, 1 % Pepton, 0,5 % NaCl) mit Tetrazyklin und Rifampicin gegeben. Positive Agrobakterien können durch Hybridisierung nachgewiesen werden.

7. Transformation von Tabak

Die Agrobakterien werden in YEB-Medium mit Rifampicin und Tetrazyclin angezüchtet. 20 ml der Bakterien werden abzentrifugiert, einmal in YEB Medium gewaschen und in 20 ml 10 mM MgSO$_4$ suspendiert in eine Petrischale gegeben. Als Pflanzenmaterial wird Nicotiana tabacum Wisconsin 38 verwendet. Die Pflanzen werden vorher 4 Wochen unter sterilen Bedingungen auf 2 MS Medium (Murashige, T. et al. Physiol. Plant 15, 473-497, 1962) bei 25°C mit 16 Std. Licht pro Tag kultiviert. Von diesen Pflanzen werden ungefähr 1 $\overline{cm}^2$ große Blattstückchen abgeschnitten, mit sterilem Schmirgelpapier verwundet und 30 sec in die Bakterienkultur eingetaucht.

Die Blattstückchen werden 2 Tage bei 25°C auf 2 MS Medium mit 0,8 % Agar gehalten und anschließend mit flüssigem 2 MS Medium mit 500 mg/l Claforan gewaschen. Danach kommen die Blattstückchen auf MSC 10 Platten. Nach 5-6 Wochen können regenerierte Pflanzen in größere Gefäße umgepflanzt werden, wo sie nach 2-3 Wochen Wurzeln bilden.

2 MS-Medium: MS-Medium mit 2 % Sucrose
MSC 10-Medium: 2 MS-Medium mit 0,1 mg/l NaA 1 mg/l BAP, 100 mg/l Kanamycin und 500 mg/l Claforan

8. Nachweis der Transformation

Aus etwa 8 Wochen alten transformierten Tabakpflanzen wird DNA isoliert, mit EcoRI gespalten und auf Nitrozellulosemembranen transferiert. Die Membran wird mit $^{32}$P-markiertem Fragment hybridisiert. Der gewünschte Einbau des Fragments in das Pflanzengenom ist dann erfolgt, wenn nach Exposition eines Röntgenfilmes das eingebaute EcoRI Fragment nachgewiesen werden kann.

9. Nachweis der Expression der RNA

Aus transgenen Tabakpflanzen wird RNA isoliert, auf einem Formaldehydgel aufgetrennt und auf eine Nitrozellulosemembran übertragen. Die Hybridisierung erfolgt mit radioaktiv markiertem Fragment.

10. Infektion mit Virus

Zum Test der Resistenz der transgenen Pflanzen wird CMV-Virus Stamm Q oder Y verwendet. Transgene Tabakpflanzen werden mit einer Virussuspension aus 10 Tage vorher infizierten Tabak- oder Gurkenpflanzen mit Hilfe von Carborundum infiziert. Transgene Pflanzen sind resistent gegen das Virus.

**Patentansprüche**

1. Gen kodierend auf RNA-Ebene für eine Sequenz, die als Initiationssequenz zur Synthese einer komplementären RNA dienen kann und die in Folge verknüpft ist mit einer antisense-Sequenz, die in sense Orientierung eine cap unabhängige Translation ermöglicht sowie mit einer antisense Sequenz, die in sense Orientierung für ein für den Stoffwechsel einer Pflanze destruktives Protein kodiert.

2. Gen nach Anspruch 1 kodierend für eine Initiationssequenz des 3'-Endes einer Virus (+)RNA oder des 3'-Endes einer Virus (-)RNA.

3. Gen nach Anspruch 1 kodierend für eine interne Initiationsstelle einer Replikase einer Virus (+)RNA oder einer Virus (-)RNA.

4. RNA kodiert durch ein Gen nach einem der Ansprüche 1 bis 3.

5. Wirtszelle enthaltend ein Gen nach einem der Ansprüche 1 bis 3.

6. Wirtszelle enthaltend eine RNA nach Anspruch 4.

7. Pflanzen, Pflanzenzellen sowie Teile oder Samen der Pflanzen, enthaltend das Gen nach einem der Ansprüche 1 bis 3.

8. Pflanzen, Pflanzenzellen sowie Teile oder Samen der Pflanzen enthaltend die RNA nach Anspruch 4.

9. Verwendung der durch das Gen nach einem der Ansprüche 1 bis 3 kodierten RNA als Vorstufe zu einem antiviralen Agens.

10. Verwendung der durch das Gen nach einem der Ansprüche 1 bis 3 kodierten RNA als antivirales Agens nach Inkubation mit Replikase.

**Patentansprüche für folgenden Vertragsstaat : ES**

1. Verfahren zur Herstellung eines Gens kodierend für eine RNA-Sequenz, dadurch gekennzeichnet, daß
a) die Synthese des ersten DNA-Teilstücks auf der Basis derjenigen Sequenz eines RNA-Virus erfolgt, die als Initiationssequenz zur Synthese einer komplementären RNA dienen kann,
b) die Synthese des zweiten DNA-Teilstücks auf der Basis einer viralen RNA erfolgt, die eine cap unabhängige Translation ermöglicht,
c) die Synthese des dritten Teilstücks auf der Basis eines Gens erfolgt, das für ein zelldestruktives Gen kodiert wobei die DNA auf RNA-Ebene für die antisense Sequenz kodiert und
die synthetisierten Oligonucleotide über Spacer miteinander verknüpft werden.

**2.** Verfahren nach Anspruch 1, dadurch gekennzeichnet, daß das Gen noch ein weiteres funktionelles Teilstück enthält.

**3.** Verfahren nach Anspruch 1, dadurch gekennzeichnet, daß die Initiationssequenz zur Synthese der Komplementären RNA so modifiziert wird, daß keine 5'-AUG-3'-Sequenzen auftreten.

**4.** Verfahren zur Transformation einer Wirtszelle, dadurch gekennzeichnet, daß das nach einem der Ansprüche 1 bis 3 erhältliche Gen mittels eines Vektors direkt oder indirekt über einen Helferstamm in die Wirtszelle gelangt.

**5.** Verwendung der RNA, die durch ein Gen kodiert wird, das durch ein Verfahren nach einem der Ansprüche 1 bis 3 kodiert wird als Vorstufe zu einem antiviralen Agens.

**6.** Verwendung der RNA, die durch ein Gen kodiert wird, das durch ein Verfahren nach einem der Ansprüche 1 bis 3 kodiert wird, nach der Inkubation mit einer Replikase als antivirales Agens.

**7.** Verfahren zur Herstellung virusresistenter Pflanzen, dadurch gekennzeichnet, daß man Pflanzenzellen mit einem Gen erhältlich nach einen der Ansprüche 1 bis 3 transformiert und zur Pflanze regeneriert.

**8.** Wirtszelle enthaltend ein Gen erhältlich nach einem der Ansprüche 1 bis 3.

**9.** Wirtszelle enthaltend eine RNA, die durch ein Gen kodiert wird, das durch ein Verfahren nach einem der Ansprüche 1 bis 3 erhältlich ist.

**10.** Pflanzen, Pflanzenzellen sowie Teile oder Samen der Pflanzen, enthaltend das Gen erhältlich nach einem der Ansprüche 1 bis 3.

**11.** Pflanzen, Pflanzenzellen sowie Teile oder Samen der Pflanzen enthaltend die RNA, die durch ein Gen kodiert wird, das durch ein Verfahren nach einem der Ansprüche 1 bis 3 erhältlich ist.

**Patentansprüche für folgenden Vertragsstaat : GR**

**1.** Gen kodierend auf RNA-Ebene für eine Sequenz, die als Initiationssequenz zur Synthese einer komplementären RNA dienen kann und die in Folge verknüpft ist mit einer antisense-Sequenz, die in sense Orientierung eine cap unabhängige Translation ermöglicht sowie mit einer antisense Sequenz, die in sense Orientierung für ein für den Stoffwechsel einer Pflanze destruktives Protein kodiert.

**2.** Gen nach Anspruch 1 kodierend für eine Initiationssequenz des 3'-Endes einer Virus ( + )RNA oder des 3'-Endes einer Virus (-)RNA.

**3.** Gen nach Anspruch 1 kodierend für eine interne Initiationsstelle einer Replikase einer Virus ( + )RNA oder einer Virus (-)RNA.

**4.** RNA kodiert durch ein Gen nach einem der Ansprüche 1 bis 3.

**5.** Wirtszelle enthaltend ein Gen nach einem der Ansprüche 1 bis 3.

**6.** Wirtszelle enthaltend eine RNA nach Anspruch 4.

**7.** Pflanzen, Pflanzenzellen sowie Teile oder Samen der Pflanzen, enthaltend das Gen nach einem der Ansprüche 1 bis 3.

**8.** Pflanzen, Pflanzenzellen sowie Teile oder Samen der Pflanzen enthaltend die RNA nach Anspruch 4.

**9.** Verwendung der durch das Gen nach einem der Ansprüche 1 bis 3 kodierten RNA als Vorstufe zu einem antiviralen Agens.

**10.** Verwendung der durch das Gen nach einem der Ansprüche 1 bis 3 kodierten RNA als antivirales Agens

11

nach Inkubation mit Replikase.